# EUROPEAN PATENT APPLICATION

(11) **EP 4 248 970 A1**
(43) Date of publication of application: **27.09.2023**
(21) Application number: 21895102.8
(22) Date of filing: 17.11.2021
(51) Int. Cl.: A61K 31/496, A61K 31/355, A61K 9/00, A61P 27/02, A61P 27/14

(54) **OPTHALMIC COMPOSITIONS COMPRISING CETIRIZINE AND TOCOFERSOLAN**

(30) Priority: 18.11.2020 KR 20200154712
(71) Applicant: Taejoon Pharmaceutical Co., Ltd., Seoul 04401 (KR)
(72) Inventor: LEE, Joon Youb, Seoul 04401 (KR); RYU, Sang Rok, Suwon-si Gyeonggi-do 16509 (KR); KIM, Han Gyeol, Suwon-si Gyeonggi-do 16498 (KR)
(74) Representative: HGF
(86) International application number: PCT/KR2021/016900
(87) International publication number: WO 2022/108334

(57) **Abstract**

An ophthalmic composition of the present invention comprises cetirizine, a pharmaceutically acceptable salt thereof and/or an optical isomer thereof as active ingredients, and comprises tocofersolan. Therefore, the ophthalmic composition of the present invention can achieve delayed release of the active ingredients, and achieve excellent stability and sensation when applied as an eye drop.

## Description

### Technical Field

The present invention relates to an ophthalmic composition comprising cetirizine and tocofersolan.

### Background

Histamine is a substance secreted as a defense reaction of the body against external stimuli, and when histamine is secreted, allergic reactions such as runny nose, tears, itching, hives, etc., occur. Cetirizine inhibits allergic symptoms by blocking histamine 1 (H1) receptors and preventing histamine from binding to the receptors, and is known as a second-generation antihistamine component used in the treatment of allergic diseases. In South Korea, a product using the component is distributed under the trade name of "Zyrtec." In addition, there is levocetirizine as one of the optical isomers known to show a main effect of blocking the H1 receptors of cetirizine. Levocetirizine is known as a third-generation antihistamine component and is distributed under the trade name "Xyzal."

Meanwhile, when the aforementioned allergic reactions occur in the eye, mast cells in the eye are activated and inflammation-inducing substances such as histamine, etc., are secreted to cause itching, redness and the like. An ophthalmic composition containing cetirizine may be administered for the prevention or treatment of such allergic eye diseases.

### Detailed Description of the Invention

### Technical Problem

One object of the present invention is to provide an ophthalmic composition capable of achieving delayed release of an active ingredient.

One object of the present invention is to provide an ophthalmic composition having improved stability.

One object of the present invention is to provide an ophthalmic composition achieving improved sensation of instillation.

### Technical Solution

An ophthalmic composition of the present invention may include cetirizine, a pharmaceutically acceptable salt thereof and/or an optical isomer thereof as active ingredients, and may include tocofersolan. The optical isomer may be levocetirizine and/or a pharmaceutically acceptable salt thereof.

In the present specification, "and/or" may be defined to refer to at least one or more of the listed components.

In the composition of the present invention, a content of the tocofersolan may be 0.1 w/v% or more, 0.3 w/v% or more, and 0.5 w/v% or more, and may be 10 w/v% or less, 5 w/v% or less, 4 w/v% or less, 3 w/v% or less, 2 w/v% or less, and 1 w/v% or less. In the composition of the present invention, the content of the tocofersolan may be 0.1 to 10 w/v%. For example, the content of the tocofersolan may be 0.1 to 5 w/v%, 0.3 to 5 w/v%, or 0.5 to 5 w/v%. For example, the content of the tocofersolan may be 0.5 w/v%, 1 w/v%, 2 w/v%, 3 w/v%, 4 w/v%, or 5 w/v%.

In the composition of the present invention, a content of the active ingredient may be 0.1 w/v% or more, 0.2 w/v% or more, 0.24 w/v% or more, 0.3 w/v% or more, and 0.36 w/v% or more, and may be 2 w/v% or less, and 1.68 w/v% or less of the composition. In the composition of the present invention, the content of the active ingredient may be 0.1 to 2 w/v%. For example, the content of the active ingredient may be 0.2 to 2 w/v%, or 0.24 to 1.68 w/v%. For example, the content of the active ingredient may be 0.24 w/v%, 0.36 w/v%, 0.48 w/v%, 0.84 w/v%, or 1.68 w/v%. The content of the active ingredient mentioned above is described based on the fact that the active ingredient is cetirizine and/or an optical isomer thereof. When the active ingredient is a pharmaceutically acceptable salt of cetirizine and/or an optical isomer thereof, it may be needless to say that the content of the active ingredient may vary depending on the type of salt. For example, 0.285 w/v% of levocetirizine hydrochloride refer to 0.24 w/v% as levocetirizine, and 0.4275 w/v% of cetirizine hydrochloride refer to 0.36 w/v% as cetirizine.

For example, the content of the tocofersolan may be 0.1 to 5 w/v%, 0.3 to 5 w/v%, or 0.5 to 5 w/v%, and the content of the active ingredient may be 0.2 to 2 w/v%, 0.3 to 2 w/v%, or 0.24 to 1.68 w/v%.

Cetirizine, a pharmaceutically acceptable salt thereof and/or an optical isomer thereof are components which cause irritation when administered to the eye, and the higher the concentration, the greater the eye irritation. The ophthalmic composition of one embodiment including tocofersolan may show excellent sensation of instillation due to less ocular discomfort such as burning sensation during instillation, even if the composition includes cetirizine, a pharmaceutically acceptable salt thereof and/or an optical isomer thereof.

The composition may exhibit a sustained effect by delaying the release of the active ingredient.

The composition may exhibit a sustained effect even with a small number of administrations by delaying the release of the active ingredient. For example, the composition may be administered once a day.

The composition may maintain the content of the active ingredient, reduce the amount of related substances, and maintain characteristics such as appearance, pH, viscosity, etc., thereby having excellent stability.

The composition may exhibit an effect of increasing efficacy of the active ingredient. For example, when the composition is administered, an anti-allergic effect and an effect of reducing allergy induction may be improved. The composition may exhibit an effect of reducing the dosage, frequency of administration, etc. of the active ingredient by increasing the efficacy of the active ingredient.

The composition may include pharmaceutically acceptable additives. For example, the composition may further include at least one of additives such as solvents, stabilizers, pH adjusters, isotonic agents, solubilizing agents, preservatives, buffers, thickeners, etc. The additives refer to a component suitable for the preparation of an ophthalmic composition.

The solvents may include an aqueous solvent. For example, the solvents may include sterile purified water, physiological saline, or water for injection.

The stabilizers may include cellulose-based compounds including carboxymethylcellulose (CMC), hydroxypropylmethylcellulose (HPMC), hydroxyethylcellulose (HEC), etc., polyvinyl-based compounds including polyvinyl alcohol (PVA), polyvinylpyrrolidone (PVP), etc., acrylic-based compounds including carbomer, etc., gums-based compounds including gellan gum, xanthan gum, etc., polysaccharides including hyaluronic acid (HA), sodium hyaluronate, sodium alginate, dextran, etc., levocarnitine, sorbitol, aminocaproic acid, ascorbic acid, vitamin E (tocopherol, α-tocopherol), vitamin E derivatives (for example, tocopheryl acetate), vitamin A (retinol), vitamin A derivatives (for example, retinyl acetate, retinyl palmitate, etc.), edetic acid and/or salts thereof (for example, sodium edetate, calcium edetate), sulfonic acid and/or salts thereof (for example, sodium sulfonate), sulfonic acid derivatives and/or salts thereof (for example, bisulfate, thiosulfate, metabisulfite, etc.), anthocyanide, flavonoid-based derivatives (for example, hesperidin, quercetin, etc.), butylhydroxytoluene (BHT), melatonin, any mixture thereof, or the like. In addition, the stabilizers may be preferably at least one selected from the group consisting of levocarnitine, aminocaproic acid, vitamin E, vitamin E derivatives, and sorbitol. Furthermore, the stabilizers may be preferably levocarnitine and aminocaproic acid. Moreover, the stabilizers may be preferably edetic acid and/or salts thereof.

The pH adjusters used herein may include sodium hydroxide, hydrochloric acid, or the like, and may be added in an amount required for obtaining an appropriate pH according to a method known to those skilled in the art.

The isotonic agents may include at least one of glycerol, mannitol, sorbitol, sodium chloride, potassium chloride, boric acid, borax, calcium chloride, sodium acetate, and sodium sulfate.

The solubilizing agents may include at least one of benzalkonium chloride, sodium lauryl sulfate, sorbitan fatty acids (sorbitan monolaurate, monopalmitate, etc.), nonoxynol 10, oxynol 9, tyloxapol, poloxamers, diethylene glycol monoethyl ether, polyethylene glycols, polyethyleneglycol sorbitan fatty acids (polysorbates 20, 40, 60, 80, etc.), polyoxyl stearic acid, polyoxyl castor oils (polyoxyl 35 castor oil, etc.), polyoxyl hydrogenated castor oils (polyoxyl 40 hydrogenated castor oil, etc.), caprylic triglycerides, capric acid triglycerides, and caprylic capric acid triglycerides.

The preservatives may include: quaternary ammonium compounds including benzalkonium chloride, benzethonium chloride, cetalkonium chloride, polyquaternium-1 (for example, poly quad), etc.; guanidine-based compounds including polyhexamethylene biguanide, chlorohexidine, etc.; chlorobutanol; mercury-based preservatives including thimerosal, phenylmercuric acetate, phenylmercuric nitrate, and the like; and antioxidants including a stabilized oxychloro complex (for example, purite), p-hydroxybenzoate alkyls (for example, methyl p-hydroxybenzoate (PM)), and the like.

The buffers may include, for example, at least one of acetic acid and/or salts thereof, citric acid and/or salts thereof, phosphoric acid and/or salts thereof (for example, sodium hydrogen phosphate and/or hydrates thereof, and sodium dihydrogen phosphate and/or hydrates thereof), boric acid and/or salts thereof, borax, carbonic acid and/or salts thereof, and arginine. An amount of the buffers to be used may be appropriately selected by those skilled in the art.

The thickeners may include: cellulose-based compounds including carboxymethyl cellulose (CMC), hydroxypropyl methylcellulose (HPMC), hydroxyethyl cellulose (HEC), etc.; polyvinyl-based compounds including polyvinyl alcohol (PVA), polyvinyl pyrrolidone (PVP), etc.; acrylic-based compounds including carbomer, etc.; gums-based compounds including gellan gum, xanthan gum, etc.; polysaccharides including hyaluronic acid (HA), sodium hyaluronate, sodium alginate, dextran, etc.; any combination thereof; or the like. In addition, the thickeners may be preferably carboxylmethyl cellulose and/or xanthan gum.

The composition may not include additives having a polyoxyethylene structure such as polyoxyl n (hydrogenated) castor oil such as polyoxyl 35 castor oil or polyoxyl 40 (hydrogenated) castor oil, polysorbate, and the like. The additives having a polyoxyethylene structure do not include tocofersolan. The composition may exhibit improved stability by including tocofersolan without including the additives having the polyoxyethylene structure. In addition, even when including the additives having the polyoxyethylene structure, the composition may exhibit excellent stability compared to an ophthalmic composition without including tocofersolan.

The composition may be a composition for an anti-allergic. The composition may be for preventing or treating allergic eye diseases. For example, the allergic eye disease may be allergic conjunctivitis.

The optical isomer may be levocetirizine or a pharmaceutically acceptable salt thereof.

The composition may be an ophthalmic composition, and specifically may be an eye drop, eye ointment, gel, etc.

In the present invention, the pharmaceutically acceptable salt means salts conventionally used in a pharmaceutical industry, and may be, for example, inorganic ion salts, inorganic acid salts, organic acid salts, sulfonic acid salts, amino acid salts, amine salts, and the like. For example, in one embodiment, the pharmaceutically acceptable salt may be hydrochloride. However, in case of the pharmaceutically acceptable salt in the present specification, the types of salts meant in the present invention may not be limited to those listed salts.

The present invention may provide an ophthalmic composition including cetirizine, a pharmaceutically acceptable salt thereof and/or an optical isomer thereof; tocofersolan; and edetic acid and/or a salt thereof.

The present invention may provide an anti-allergic method including administering an ophthalmic composition containing cetirizine, a pharmaceutically acceptable salt thereof and/or an optical isomer thereof as active ingredients and containing tocofersolan to a subject in need of treatment, and the present invention may provide a method for preventing or treating allergic eye diseases, which includes administering an ophthalmic composition containing cetirizine, a pharmaceutically acceptable salt thereof and/or an optical isomer thereof as active ingredients and containing tocofersolan to a subject in need of treatment. As described above, the allergic eye disease may be allergic conjunctivitis.

In the present invention, the "subject" may refer to all the animals including humans, who have been or are likely to be diagnosed with allergic eye diseases. The animals may include not only humans, but also mammals such as cow, horse, sheep, pig, goat, camel, antelope, dog, cat, etc., which need a treatment for allergic eye diseases or symptoms similar thereto, but are not limited thereto.

In the present invention, the "administration" may refer to introducing the ophthalmic composition of the present invention into patients through any appropriate method, and an administration route of the present invention may refer to being locally administered to eyes, if the composition is an eye drop. The method for treating allergic eye diseases according to the present invention may include administering the ophthalmic composition of the present invention in a therapeutically effective amount.

The present invention may provide a use of the ophthalmic composition for an anti-allergic including cetirizine, the pharmaceutically acceptable salt thereof and/or the optical isomer thereof as active ingredients, and including tocofersolan.

The present invention may provide a use of the ophthalmic composition including cetirizine, the pharmaceutically acceptable salt thereof and/or the optical isomer thereof as active ingredients, and including tocofersolan for the manufacture of a medicament for an anti-allergic.

The present invention may provide a use of the ophthalmic composition including cetirizine, the pharmaceutically acceptable salt thereof and/or the optical isomer thereof as active ingredients, and including tocofersolan for preventing or treating allergic eye diseases. As described above, the allergic eye disease may be allergic conjunctivitis.

The present invention may provide a use of the ophthalmic composition including cetirizine, the pharmaceutically acceptable salt thereof and/or the optical isomer thereof as active ingredients, and including tocofersolan for the manufacture of a medicament for preventing or treating allergic eye diseases. As described above, the allergic eye disease may be allergic conjunctivitis.

The present invention may provide a method for stabilizing cetirizine, a pharmaceutically acceptable salt thereof and/or an optical isomer thereof, which includes a step for mixing cetirizine, a pharmaceutically acceptable salt thereof and/or an optical isomer thereof, and tocofersolan.

The present invention may provide a method for preparing a composition, which includes a step for mixing cetirizine, a pharmaceutically acceptable salt thereof and/or an optical isomer thereof, and tocofersolan. The preparation method may further include a step for mixing additives. In addition, the method may further include a step for a filtering or high-temperature and highpressure sterilization process in order to sterilize the composition.

Regarding the ophthalmic composition according to the present invention, the use of the compositions, the method for stabilizing cetirizine, the pharmaceutically acceptable salt thereof and/or the optical isomer thereof, the method for preparing the composition, and the method for prevention or treatment including a step for administering the compositions, descriptions applied to the above compositions, the use thereof, the preparation method thereof or the methods may be equally applied to the respective compositions, uses or methods, if not contradictory to each other.

### Advantageous Effects

An ophthalmic composition according to one embodiment of the present invention can achieve delayed release of the active ingredient. An ophthalmic composition according to one embodiment of the present invention can exhibit sustained efficacy due to the delayed release of the active ingredient. In addition, the present invention can provide an ophthalmic composition with increased efficacy, and can exhibit an effect of reducing the dosage and/or frequency of administration. Furthermore, an ophthalmic composition according to one embodiment of the present invention can have excellent stability such as suppression of formation of related substances such as N-Oxide, and alleviate side effects such as burning sensation in the eye, thereby providing improved sensation of instillation.

### Brief Description of the Drawings

FIG. 1 is a graph of showing the results of identifying a release delay effect of Experimental Example 1.
FIG. 2 is a graph of showing the results (the amount of N-oxide produced) of identifying a stability improvement effect of Experimental Example 2.
FIG. 3 is a graph of showing the results of identifying a release delay effect of Experimental Example 4.
FIG. 4 is a graph of showing the results of identifying a release delay effect of Experimental Example 5.
FIG. 5 is a graph of showing the results of identifying a release delay effect of Experimental Example 6.
FIG. 6 is a graph of showing the results of identifying an effect of improving sensation of instillation (evaluating scores of burning sensation) of Experimental Example 7.
FIG. 7 is a graph of showing the results of identifying an effect of inhibiting allergic reactions of Experimental Example 8.

### Mode for Invention

Hereinafter, the present invention will be described in detail through preferred Examples for better understanding of the present invention. However, the following Examples are provided only for the purpose of illustrating the present invention, and thus the present invention is not limited thereto.

### Experimental Example 1: Confirmation of release delay effect

Compositions were prepared in accordance with components and contents as shown in table 1 below. Specifically, composition 1 was prepared by adding and dissolving sodium hydrogen phosphate anhydrous and levocetirizine hydrochloride in sterile purified water at a high temperature (for example, 50°C or higher). Composition 2 was prepared by adding and dissolving sodium hydrogen phosphate anhydrous, levocetirizine hydrochloride and tocofersolan in sterile purified water at a high temperature (for example, 50°C or higher). The pH of the prepared compositions was adjusted to about 6.5, which were then sterilized.

**[Table 1]**

| Component and Content (mg/ml) | Composition 1 | Composition 2 |
|---|---|---|
| Levocetiiizine hydrochloride | 2.85 | 2.85 |
| Tocofersolan | - | 5 |
| Sodium hydrogen phosphate anhydrous | 2 | 2 |

Prepared compositions 1 and 2 were put into a semi-permeable membrane (Float A lyzer) and then put into a dissolution tester (SOTAX^{™}) containing a simulated tear fluid (STF) solution, so as to evaluate an amount of an active ingredient released over time by using a liquid chromatogram.

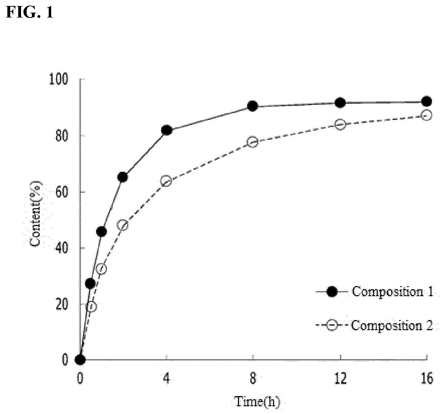

As a result, as shown in FIG. 1, it was confirmed for composition 2 that the release of the active ingredient is remarkably delayed compared to composition 1. Accordingly, it may be seen that the release of levocetirizine is effectively delayed in the ophthalmic composition of one example including tocofersolan.

### Experimental Example 2: Confirmation of stability improvement effect

Compositions were prepared in accordance with components and contents as shown in table 2 below. Specifically, composition 3 was prepared by adding and dissolving polyoxyl 35 castor oil, sodium hydrogen phosphate anhydrous and levocetirizine hydrochloride in sterile purified water at a high temperature (for example, 50°C or higher). Composition 4 was prepared by adding and dissolving polyoxyl 35 castor oil, sodium hydrogen phosphate anhydrous, levocetirizine hydrochloride and tocofersolan in sterile purified water at a high temperature (for example, 50°C or higher). The pH of the prepared compositions was adjusted to about 6.5, which were then sterilized.

**[Table 2]**

| Component and Content (mg/ml) | Composition 3 | Composition 4 |
|---|---|---|
| Levocetirizine hydrochloride | 2.85 | 2.85 |
| Polyoxyl 35 Castor Oil | 10 | 10 |
| Tocofersolan | - | 5 |
| Sodium hydrogen phosphate anhydrous | 2 | 2 |

After storing the prepared compositions at 70°C for seven days, an amount of levocetirizine N-oxide produced was measured by using a liquid chromatogram.

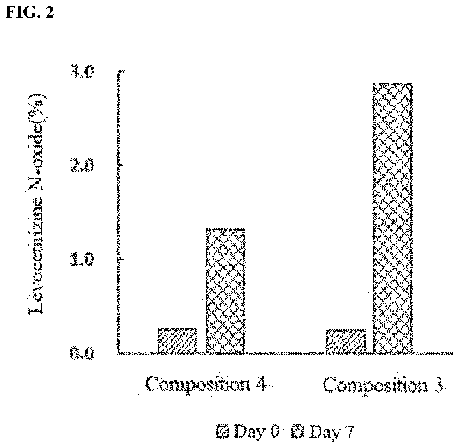

As a result, as shown in FIG. 2, it was confirmed that the amount of N-Oxide produced from composition 4 including tocofersolan is much smaller than that of composition 3 without including tocofersolan. Accordingly, it was confirmed that tocofersolan improves the stability of the cetirizine composition, and it can be seen that the stability of the composition of one example including tocofersolan is excellent.

### Experimental Example 3: Confirmation of effect of improving sensation of instillation

Compositions were prepared in accordance with components and contents as shown in table 3 below. Specifically, composition 5 was prepared by adding and dissolving sodium hydrogen phosphate anhydrous, levocarnitine, sodium chloride and levocetirizine hydrochloride in sterile purified water at a high temperature (for example, 50°C or higher). Compositions 6 to 10 were prepared by adding and dissolving sodium hydrogen phosphate anhydrous, levocarnitine, sodium chloride, levocetirizine hydrochloride and tocofersolan in sterile purified water at a high temperature (for example, 50°C or higher). The pH of the prepared compositions was adjusted to about 6.5, which were then sterilized. An osmolality of the prepared compositions was about 290 mOsmol/kg.

**[Table 3]**

| Component and Content (mg ml) | Composition 5 | Composition 6 | Composition 7 | Composition a | Composition 9 | Composition 10 |
|---|---|---|---|---|---|---|
| Levocetirizine hydrochloride | 9.95 | 9.98 | 9.98 | 9.98 | 9.98 | 9.98 |
| Tocofereolan | - | 10 | 20 | 30 | 40 | 50 |
| Levocarnitine | 2.5 | 2.5 | 2.5 | 2.5 | 2.5 | 2.5 |
| Sodium hydrogen phosphorus | 2 | 2 | 2 | 2 | 2 | 2 |
| Sodium chloride | Suitable amount | Suitable amount | Suitable amount | Suitable amount | Suitable amount | Suitable amount |

The prepared compositions were instilled on both eyes of adults and the sensation of instillation was evaluated for about three minutes.

As a result, it was confirmed that composition 5 without including tocofersolan is unsuitable as an eye drop by causing very severe burning sensation, and it was confirmed that slight burning sensation is felt or no burning sensation is felt when compositions 6 to 10 including tocofersolan are administered (Table 4). Although the compositions include a high concentration of levocetirizine, it was confirmed that the sensation of instillation is remarkably improved depending on the concentration of tocofersolan. Accordingly, it can be seen that tocofersolan remarkably ameliorates burning sensation induced by cetirizine.

**[Table 4]**

| Composition | Burning sensation |
|---|---|
| 5 | Very severe burning sensation |
| 6 | Slight burning sensation |
| 7 | Almost none |
| 8 | None |
| 9 | None |
| 10 | None |

### Experimental Example 4: Confirmation of release delay effect

Compositions were prepared in accordance with components and contents as shown in table 5 below. Specifically, composition 11 was prepared by adding and dissolving buffer, glycerin and levocetirizine hydrochloride in sterile purified water at a high temperature (for example, 50°C or higher). Compositions 12 and 13 were prepared by adding and dissolving buffer, glycerin, levocetirizine hydrochloride and tocofersolan in sterile purified water at a high temperature (for example, 50°C or higher). The pH of the prepared compositions was adjusted to about 6.5, which were then sterilized. An osmolality of the prepared compositions was about 290 mOsmol/kg.

**[Table 5]**

| Component and Content (mg/ml) | Composition 11 | Composition 12 | Composition 13 |
|---|---|---|---|
| Levocetirizine hydrochloride | 1.187 | 1.187 | 1.187 |
| Tocofersolan | - | 3 | 5 |
| Buffer | Suitable amount | Suitable amount | Suitable amount |
| Glycerin | Suitable amount | Suitable amount | Suitable amount |

Prepared compositions 11 to 13 were put into a semi-permeable membrane (Float A lyzer) and then put into a dissolution tester (SOTAX^{™}) containing a simulated tear fluid (STF) solution, so as to evaluate an amount of the active ingredient released over time by using a liquid chromatogram.

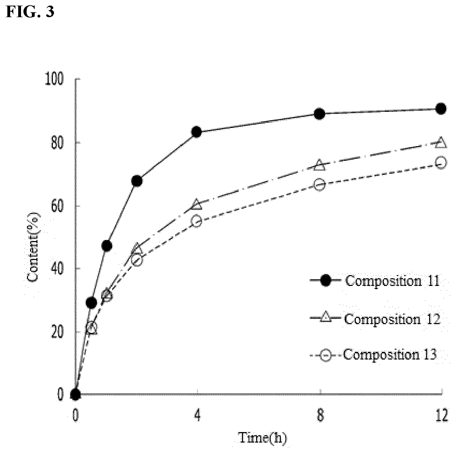

As a result, as shown in FIG. 3, it was confirmed for compositions 12 and 13 that the release of the active ingredient is remarkably delayed compared to composition 11. Accordingly, it may be seen that the release of levocetirizine is effectively delayed from the ophthalmic composition including tocofersolan.

### Experimental Example 5: Confirmation of release delay effect

Compositions were prepared in accordance with components and contents as shown in table 6 below. Specifically, composition 14 was prepared by adding and dissolving buffer and levocetirizine hydrochloride in sterile purified water at a high temperature (for example, 50°C or higher). Compositions 15 and 16 were prepared by adding and dissolving buffer, levocetirizine hydrochloride and tocofersolan in sterile purified water at a high temperature (for example, 50°C or higher). The pH of the prepared compositions was adjusted to about 6.5, which were then sterilized.

**[Table 6]**

| Component and Content (mg/ml) | Composition 14 | Composition 15 | Composition 16 |
|---|---|---|---|
| Levocetirizine hydrochloride | 23.75 | 23.75 | 23.75 |
| Tocofersolan | - | 20 | 100 |
| Buffer | Suitable amount | Suitable amount | Suitable amount |

Prepared compositions 14 to 16 were put into a semi-permeable membrane (Float A lyzer) and then put into a dissolution tester (SOTAX^{™}) containing a simulated tear fluid (STF) solution, so as to evaluate an amount of the active ingredient released over time by using a liquid chromatogram.

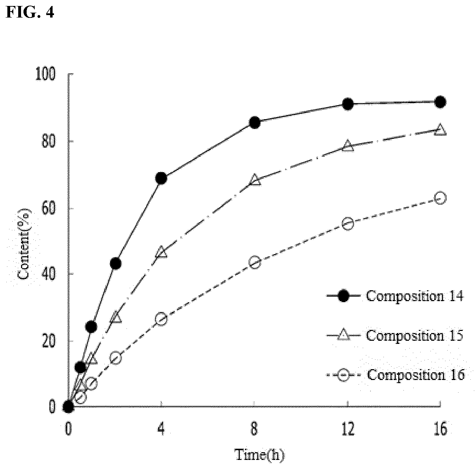

As a result, as shown in FIG. 4, it was confirmed for compositions 15 and 16 that the release of the active ingredient is remarkably delayed compared to composition 14. Accordingly, it may be seen that the release of levocetirizine is effectively delayed from the ophthalmic composition including tocofersolan.

### Experimental Example 6: Confirmation of release delay effect

Compositions were prepared in accordance with components and contents as shown in table 7 below. Specifically, composition 17 was prepared by adding and dissolving buffer, glycerin and cetirizine hydrochloride in sterile purified water at a high temperature (for example, 50°C or higher). Composition 18 was prepared by adding and dissolving buffer, glycerin, cetirizine hydrochloride and tocofersolan in sterile purified water at a high temperature (for example, 50°C or higher). The pH of the prepared compositions was adjusted to about 6.5, which were then sterilized. An osmolality of the prepared compositions was about 290 mOsmol/kg.

**[Table 7]**

| Component and Content (mg/ml) | Composition 17 | Composition 18 |
|---|---|---|
| Cetirizine hydrochloride | 4.275 | 4.275 |
| Tocofersolan | - | 10 |
| Buffer | Suitable amount | Suitable amount |
| Glycerin | Suitable amount | Suitable amount |

Prepared compositions 17 and 18 were put into a semi-permeable membrane (Float A lyzer) and then put into a dissolution tester (SOTAX^{™}) containing a simulated tear fluid (STF) solution, so as to evaluate an amount of the active ingredient released over time by using a liquid chromatogram.

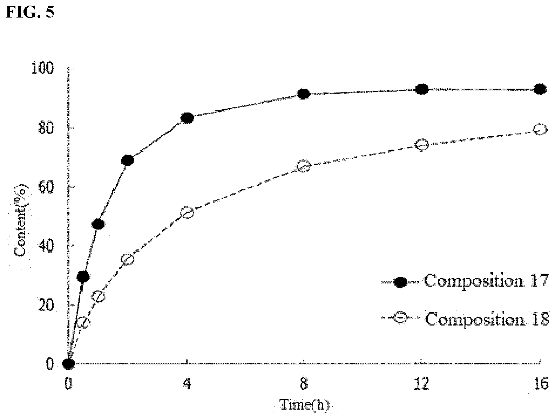

As a result, as shown in FIG. 5, it was confirmed for composition 18 that the release of the active ingredient is remarkably delayed compared to composition 17. Accordingly, it can be seen that the release of cetirizine is effectively delayed from the composition including tocofersolan.

### Experimental Example 7: Confirmation of effect of improving sensation of instillation

Compositions were prepared in accordance with components and contents as shown in table 8 below. Specifically, compositions 19 and 21 were prepared by adding and dissolving sodium edetate hydrate, glycerin, buffer, benzalkonium chloride and levocetirizine hydrochloride or cetirizine hydrochloride in sterile purified water at a high temperature (for example, 50°C or higher) and then adding and hydrating xanthan gum. Compositions 20 and 22 were prepared by adding and dissolving sodium edetate hydrate, glycerin, buffer, benzalkonium chloride, levocetirizine hydrochloride or cetirizine hydrochloride and tocofersolan in sterile purified water at a high temperature (for example, 50°C or higher) and then adding and hydrating xanthan gum. The pH of the prepared compositions was adjusted to about 6.5, which were then sterilized. An osmolality of the prepared compositions was about 290 mOsmol/kg.

**[Table 8]**

| Component and Content (mg/ml) | Composition 19 | Composition 20 | Composition 21 | Composition 22 |
|---|---|---|---|---|
| Levocetirizine hydrochloride | 4.75 | 4.75 | - | - |
| Cetirizine hydrochloride | - | - | 2.85 | 2.85 |
| Tocofersolan | - | 10 | - | 5 |
| Sodium edetate hydrate | 0.25 | 0.25 | 0.25 | 0.25 |
| Glycerin | Suitable amount | Suitable amount | Suitable amount | Suitable amount |
| Buffer | Suitable amount | Suitable amount | Suitable amount | Suitable amount |
| Xanthan gum | 1.7 | 1.7 | 1.7 | 1.7 |
| Benzalkonium chloride | 0.1 | 0.1 | 0.1 | 0.1 |

The prepared compositions were instilled on both eyes of ten adults and a score of burning sensation was evaluated for three minutes according to the criteria of table 9 below.

**[Table 9]**

| | |
|---|---|
| 0 | No unpleasant feeling and very soft |
| 1-2 | Slightly burning feeling |
| 3-4 | Slight pain with burning sensation |
| 5 | Burning pain persists, making daily life almost impossible |

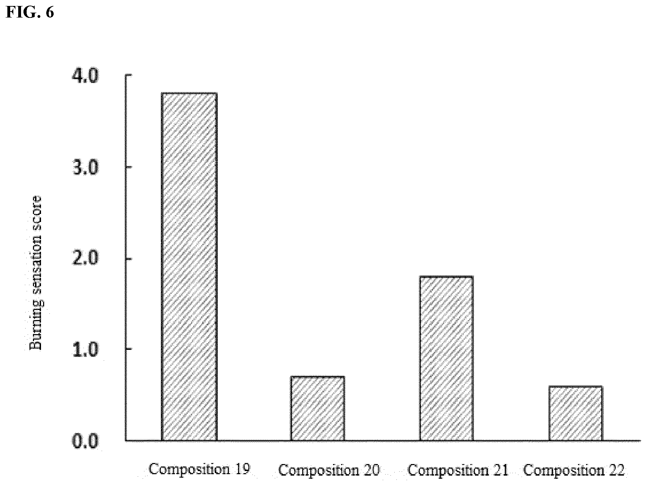

As a result, it was confirmed that compositions 19 and 21 without including tocofersolan cause burning sensation, while burning sensation is hardly felt when compositions 20 and 22 including tocofersolan are administered (FIG. 6). Accordingly, it can be seen that tocofersolan remarkably ameliorates burning sensation induced by cetirizine and levocetirizine.

### Experimental Example 8: Confirmation of inhibitory effect on allergic reaction

Compositions were prepared in accordance with components and contents as shown in table 10 below. Specifically, the compositions were prepared by adding and dissolving sodium edetate hydrate, glycerin, buffer, levocarnitine, aminocaproic acid, benzalkonium chloride, levocetirizine hydrochloride (compositions 23 and 24) and tocofersolan (compositions 24 and 25) in sterile purified water at a high temperature (for example, 50°C or higher) and then adding and hydrating xanthan gum. The pH of the prepared compositions was adjusted to about 6.5, which were then sterilized. An osmolality of the prepared compositions was about 290 mOsmol/kg.

**[Table 10]**

| Component and Content (mg/ml) | Composition 23 | Composition24 | Composition 2.5 |
|---|---|---|---|
| Levocetirizine hydrochloride | 2.85 | 2.85 | - |
| Tocofersolan | - | 5.0 | 5.0 |
| Sodium edetate hydrate | 0.25 | 0.25 | 0.25 |
| Glycerin | Suitable amount | Suitable amount | Suitable amount |
| Buffer | Suitable amount | Suitable amount | Suitable amount |
| Xanthan gum | 1.7 | 1.7 | 1.7 |
| Levocarnitine | 2.5 | 2.5 | 2.5 |
| Aminocaproic acid | 2.0 | 2.0 | 2.0 |
| Benzalkonium chloride | 0.1 | 0.1 | 0.1 |

The prepared compositions 23 to 25 were instilled into eyes in an amount of 100 µL, respectively, 4 and 16 hours before administering histamine to New Zealand White Rabbits. Evans blue solution was intravenously administered one hour before administering histamine. One hour after administering the Evans blue solution, the animals were anesthetized and subconjunctivally dosed with histamine, and then necropsy was performed 30 minutes later so as to measure a concentration of Evans blue in the conjunctival tissue.

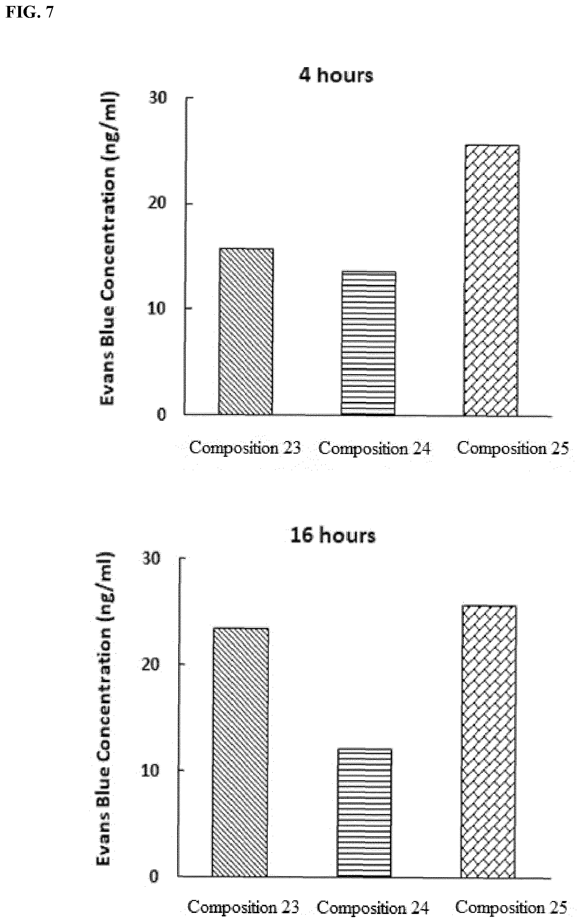

As a result, it was confirmed that an anti-allergic effect is excellent in the group dosed with composition 24 even when histamine is administered 16 hours later (FIG. 7). Accordingly, it can be seen that the composition including tocofersolan has an excellent effect of persistently inhibiting allergic reactions.

Referring to Experimental Examples 1 to 8 described above, it was confirmed that the ophthalmic composition including cetirizine, the pharmaceutically acceptable salt thereof and/or the optical isomer thereof as active ingredients and including tocofersolan according to the present invention exhibits excellent stability and sensation of instillation while achieving delayed release.

While the present invention has been described in detail above, it is apparent to those skilled in the art that such detailed descriptions are set forth to illustrate exemplary embodiments only, but are not construed to limit the scope of the present invention. Thus, it should be understood that the substantial scope of the present invention is defined by the accompanying claims and equivalents thereto.

## Claims

1. An ophthalmic composition comprising cetirizine, a pharmaceutically acceptable salt thereof, an optical isomer thereof or a mixture of at least two thereof as an active ingredient, and comprising tocofersolan.

2. The ophthalmic composition of claim 1, wherein a content of the tocofersolan is 0.1 to 10 w/v%.

3. The ophthalmic composition of claim 1, wherein a content of the active ingredient is 0.1 to 2 w/v%.

4. The ophthalmic composition of claim 1, wherein the composition further comprises at least one of solvents, stabilizers, pH adjusters, isotonic agents, preservatives, solubilizing agents, thickeners, and buffers.

5. The ophthalmic composition of claim 1, wherein the composition is a composition for an anti-allergic.

6. The ophthalmic composition of claim 1, wherein the composition is for preventing or treating allergic conjunctivitis.

7. The ophthalmic composition of claim 1, wherein the optical isomer is levocetirizine or a pharmaceutically acceptable salt thereof.

8. The ophthalmic composition of claim 1, wherein the composition is an eye drop.

9. A method for preventing or treating allergic conjunctivitis, the method comprising administering the ophthalmic composition of any one of claims 1 to 4, 7 and 8 into a subject.

10. A use of the ophthalmic composition of any one of claims 1 to 4, 7 and 8 for the manufacture of a medicament for preventing or treating allergic conjunctivitis.

11. A use of the ophthalmic composition of any one of claims 1 to 4, 7 and 8 for preventing or treating allergic conjunctivitis.
